# FASCICULE DE BREVET EUROPEEN

(11) **EP 0 742 821 B1**
(45) Date de publication et mention de la délivrance du brevet: **19.04.2006**
(21) Numéro de dépôt: 95908302.3
(22) Date de dépôt: 02.02.1995
(51) Int. Cl.: C12N 15/10, C12N 15/62, C12N 7/01, C07K 16/10

(54) **PROCEDE DE PREPARATION D'UNE BANQUE MULTICOMBINATOIRE DE VECTEURS D'EXPRESSION DE GENES D'ANTICORPS**
HERSTELLUNG EINER MULTIKOMBINATORISCHEN BANK VON ANTIKÖRPERGENE-EXPRIMIERENDE VEKTOREN
METHOD FOR PREPARING A MULTICOMBINATORIAL LIBRARY OF ANTIBODY GENE EXPRESSION VECTORS

(30) Priorité: 10.02.1994 FR 9401519
(43) Date de publication de la demande: 20.11.1996
(73) Titulaire: Sanofi Pasteur, 69367 Lyon Cedex 07 (FR)
(72) Inventeur: SODOYER, Régis, F-69110 Sainte-Foy-lès-Lyon (FR); AUJAME, Luc, F-69210 Fleurieux-sur-l'Arbresle (FR); GEOFFROY, Frédérique, F-69690 Bessenay (FR)
(74) Mandataire: Bernasconi, Jean Raymond
(86) Numéro de dépôt international: PCT/FR1995/000127
(87) Numéro de publication internationale: WO 1995/021914

(56) Documents cités:
- WO-A-92/20791
- WO-A-93/19172
- NUCL. ACIDS RES., vol. 21, IRL PRESS, OXFORD, ENGLAND;, page 2265 P. WATERHOUSE 'Combinatorial infection and in vivo recombination: a strategy for making large phage antibody repertoires' cité dans la demande
- BIO/TECHNOLOGY, vol. 2, NATURE AMERICA, INC., NEW YORK, US;, page 1045 K. BACKMAN ET AL. 'Use of synchronous site-specific recombination in vivo to regulate gene expression'
- PROC. NATL. ACAD SCI., vol. 89, NATL. ACAD SCI., WASHINGTON, DC, US;, page 1026 T.A. COLLET ET AL. 'A binary plasmid system for shuffling combinatorial antibody libraries'
- EMBO J., vol. 13,no. 14, 15 Juillet 1994 OXFORD UNIVERSITY PRESS,GB;, pages 3245-3260, A.D. GRIFFITH ET AL. 'Isolation of high affinity human antibodies directly from large synthetic repertoires'
- GENE (AMSTERDAM) 151 (1-2). 1994. 109-113. ISSN: 0378-1119, GEOFFROY F ET AL 'A new phage display system to construct multicombinatorial libraries of very large antibody repertoires.'

## Description

Les molécules d'anticorps sont constituées par l'association de deux chaînes lourdes (H) et de deux chaînes légères (L) par l'intermédiaire de ponts disulfure. Les deux chaînes lourdes sont associées entre elles selon une structure en forme de Y et les deux chaînes légères s'associent respectivement aux deux branches de cette structure, de façon telle que les domaines variables des chaînes légères (V_{L}) et lourdes (V_{H}) soient situés à proximité l'un de l'autre. La liaison à l'antigène résulte des propriétés des parties variables des chaînes légères et lourdes. Un système complexe de réarrangement et de sélection permet alors d'induire rapidement une quantité importante d'anticorps spécifiques contre un antigène.

La technique classique des hybridomes permet la sélection de clones de cellules hybrides exprimant des gènes codants pour les chaînes légère et lourde d'une molécule d'anticorps. Cette technique demande la fusion de cellules d'origine lymphocytaire, contenant les gènes présidant à la formation des anticorps et de cellules susceptibles de conduire à des hybridomes formant des lignées immortalisées. Les cellules portant les gènes en question sont en général obtenues par création au hasard de banques de cellules circulantes, avec ou sans immunisation préalable par l'antigène spécifique, et le criblage des hybridomes s'effectue par une réaction antigène-anticorps après multiplications et cultures des clones hybridomes. Cette technique est lourde, d'un rendement limité et le criblage n'est pas facile.

Une autre méthode, utilisant des bactériophages recombinants a été récemment mise en oeuvre. Les principes et diverses réalisations de ce procédé sont par exemple décrits par D. R. Burton, Tiptech - mai 1991, vol. 9, 169-175 ; D. J. Chiswell et al., Tiptech - mars 1992, vol. 10, 80-84 ; H. R. Hoogenboom et al., Rev. Fr. Transfus. Hémobiol., 1993, 36, 19-47 (voir également les demandes de brevets PCT WO 92/01047 et 92/20791).

Cette technique consiste à insérer, dans un vecteur, un répertoire de gènes de régions variables d'anticorps en association avec un gène de bactériophage dans des conditions permettant l'expression du gène sous la forme d'une protéine de fusion se présentant à la surface du phage, exposant les régions variables des chaînes variables légère et lourde associées par leurs ponts disulfure à la façon d'un fragment Fab d'anticorps, et à sélectionner directement les phages par une méthode rapide de séparation utilisant l'antigène spécifique immobilisé, par exemple par chromatographie d'immuno-affinité. Les phages sélectionnés, après élution, peuvent infecter une bactérie et être utilisés pour une, production directe ou pour répéter des cycles de sélection. Cette méthode est particulièrement puissante car elle permet la création, en théorie, de banques très importantes et un criblage extrêmement fin, efficace et rapide de la banque. Un phage qui se prête particulièrement bien à ce procédé est le phage filamenteux fd, dans lequel 1 on peut fusionner le fragment codant pour l'une des chaînes lourde et légère de l'anticorps avec le gène de la protéine mineure de surface et insérer le fragment codant pour l'autre chaîne, de sorte que, après infection de bactéries par le phage, on obtient une population de phages portant à leur surface une protéine de fusion présentant les chaînes lourde et légère dans une configuration capable de reconnaître l'antigène, et donc criblable.

Outre sa simplicité, cette technique présente de grands avantages. En association avec l'amplification préalable de la banque de gènes d'anticorps, on peut arriver à sélectionner un phage présentant un fragment d'anticorps spécifique dans une très grande population de phages, de l'ordre de 10⁷, ce qui peut permettre de rechercher les gènes d'anticorps humains sans avoir forcément à procéder à l'immunisation préalable du donneur.

Par clivage suivi de religation, ou à partir de deux banques séparées de gènes de chaînes légères et de chaînes lourdes, on peut obtenir des phages associant une chaîne légère et une chaîne lourde au hasard.

Le nombre de clones différents que l'on peut obtenir est cependant limité par le rendement de la sélection et par le degré d'efficacité de la transformation bactérienne.

Un moyen d'accroître le nombre d'associations réussies associant les chaînes légères d'une première banque aux chaînes lourdes d'une deuxième banque a été décrit par **P**. **WATERHOUSE** et al., Nucleic acids Research, 1993, Vol. 21, No. 9, 2265-2266, permettant d'obtenir jusqu'à 10¹² clones, en utilisant un système de recombinaison spécifique de sites loxP sensible à l'action d'une recombinase Cre. Cependant l'association reste réversible. De plus 11 n'existe aucun contrôle de l'action de la recombinase et les vecteurs recombinés n'ont aucun avantage sélectif par rapport aux autres vecteurs. La demande de brevet PCT WO 93/19172 décrit un procédé de préparation d'une banque multicombinatoire de membres d'une paire de liaison, notamment par intégration réversible d'un phagemide portant un site Att P dans un plasmide présentant un site Att B, l'ADN intégré étant alors flanqué de deux sites AttL et AttR.

Or, compte-tenu du rendement de l'étape de sélection des phages recombinés, qui, en réalité, ne retient qu'une fraction des phages intéressants, il est souhaitable d'obtenir les plus hauts rendements possibles de vecteurs recombinés avec le moins possible de vecteurs non-recombinés.

Un objectif de l'invention est donc de fournir un procédé de production de banques multicombinatoires, et notamment sous forme de phages ou phagemides, à partir de deux répertoires de gènes, l'un de chaînes légères et l'autre de chaînes lourdes, permettant d'obtenir un nombre de clones très élevé.

Un autre objectif de l'invention est de fournir un tel procédé dans lequel le nombre de vecteurs non-recombinés présents à la fin du procédé soit réduit.

Un autre objectif de l'invention est de produire de tels recombinants ayant une stabilité accrue.

L'invention a pour objet un procédé de production de banques multicombinatoires dans lequel, partant d'un premier répertoire de gènes codant pour une population d'un de deux types de polypeptides susceptibles de s'associer, de manière covalente ou non, notamment de régions variables de l'un des types chaîne légère et chaîne lourde d'anticorps, et d'au moins d'un gène codant pour l'autre type de polypeptide, notamment une région variable de l'autre type, de chaîne d'anticorps ou de préférence d'un second répertoire de gènes codants pour une population dudit autre type, on introduit les gènes dudit premier répertoire dans un premier vecteur pour former une population de vecteurs portant les différents gènes dudit premier répertoire, et on introduit ledit gène dudit autre type ou les gènes dudit second répertoire dans un second vecteur, l'un au moins desdits vecteurs de type phagemide, dit receveur, étant agencé pour recevoir, par recombinaison avec l'autre vecteur de type plasmide, tout ou partie dudit autre vecteur, avec son gène, dans des conditions permettant audit vecteur phagemide receveur de contenir, après recombinaison, un gène de l'un des deux types et un gène de l'autre type et d'exprimer les deux gènes sous forme de polypeptides associés susceptibles d'apparaître à la surface extérieure d'un phage en y restant maintenus et en étant associés entre eux de façon multimérique ou simulant un multimère.

Par la suite l'invention sera décrite dans l'application dans laquelle les deux types de polypeptides sont des régions, au moins en partie variables, de chaînes légères et de chaînes lourdes d'anticorps. Cependant on comprend qu'elle s'applique à d'autres types de polypeptides susceptibles de s'associer, et notamment les chaînes de récepteurs hétérodimériques, tels que les chaînes α et β ou γ et δ des récepteurs des cellules T.

Les vecteurs, de préférence circularisés, contiennent respectivement les sites de recombinaison spécifiques attB de E. coli et attP du phage λ agencés de façon à permettre la recombinaison sous l'effet de la recombinase ou intégrase associée en formant de façon irréversible, dans un vecteur unique résultant de la recombinaison, des séquences de jonction stables attL et attR.

Bien entendu on pourrait également remplacer ces sites par des sites de recombinaison spécifiques d'autres systèmes de recombinaison, bacterie-phage ou autres.

De façon à permettre l'élimination des vecteurs ne comportant qu'une chaîne unique et obtenir en conséquence un accroissement supplémentaire de la teneur relative en vecteurs recombinés, ledit vecteur peut être agencé pour présenter un marqueur de sélection initialement non fonctionnel et rendu fonctionnel par la recombinaison.

De préférence le marqueur de sélection comporte un gène permettant la sélection, lorsqu'il est exprimé, et un promoteur propre au gène, le promoteur étant inséré dans l'un des vecteurs et le gène marqueur dans l'autre, de façon à se retrouver associés dans le vecteur de recombinaison obtenu. Parmi les marqueurs préférés figurent les gènes de résistance, avec leur promoteur, au chloramphenicol, aux tétracyclines et à la gentanycine.

Bien entendu les deux vecteurs peuvent également comporter des marqueurs propres utilisables dans la construction spécifique de chacun des vecteurs, de façon à permettre ou améliorer la sélection de chacun des vecteurs pendant son processus de construction. A titre d'exemple ces marqueurs peuvent être les gènes de résistance à des antibiotiques tels que la kanamycine et l'ampicilline.

De façon préférée on utilise, pour contrôler l'étape de recombinaison entre le premier et le second vecteur, une recombinase thermo-inductible, par exemple par le choix d'un hôte approprié tel que la souche E. coli D1210 HP figurant au catalogue de la société américaine Stratagène.

L'invention a également pour objet les vecteurs phagemides susceptibles d'être obtenus par le procédé selon l'invention, et qui se caractérisent par la présence d'une séquence codant pour une partie variable de chaîne légère d'anticorps, et une séquence codant pour une partie variable de chaîne lourde d'anticorps, lesdites séquences étant accompagnées, dans des cadres convenables, des éléments permettant leur expression dans un hôte, lesdites séquences de chaîne légère et de chaîne lourde, avec lesdits moyens qui leur sont associés, étant respectivement séparées par des sites uniques d'intégration irreversibles att R et att L.

Enfin l'invention a pour objet les banques multicombinatoires, formées par les vecteurs selon l'invention et associant, de façon aléatoire, une séquence codant pour l'un des types de polypeptides, tel qu'une partie variable de chaîne légère et une séquence codant pour l'autre type de polypeptide tel qu'une partie variable de chaîne lourde.

On va maintenant décrire un exemple de construction d'un vecteur recombiné selon l'invention combinant les parties variables de la chaîne légère et de la chaîne lourde d'un même clone anti-gp160 de HIV, vecteur qui s'avère capable d'exprimer les gènes desdites parties variables légères et lourde et de présenter leurs produits d'expression à sa surface ou, en variante, de les relarguer sous forme d'une association chaîne lourde-chaîne légère reconnaissant l'antigène gp160.

La même technique pourra être utilisée pour réaliser les constructions multicômbinàtoires associant les gènes d'un répertoire de parties variables de chaîne légère à un gène de partie variable de chaîne lourde, ou un répertoire de parties variables de chaîne lourde à un gène de partie variable de chaîne légère, ou deux répertoires de parties variables lourdes et légères.

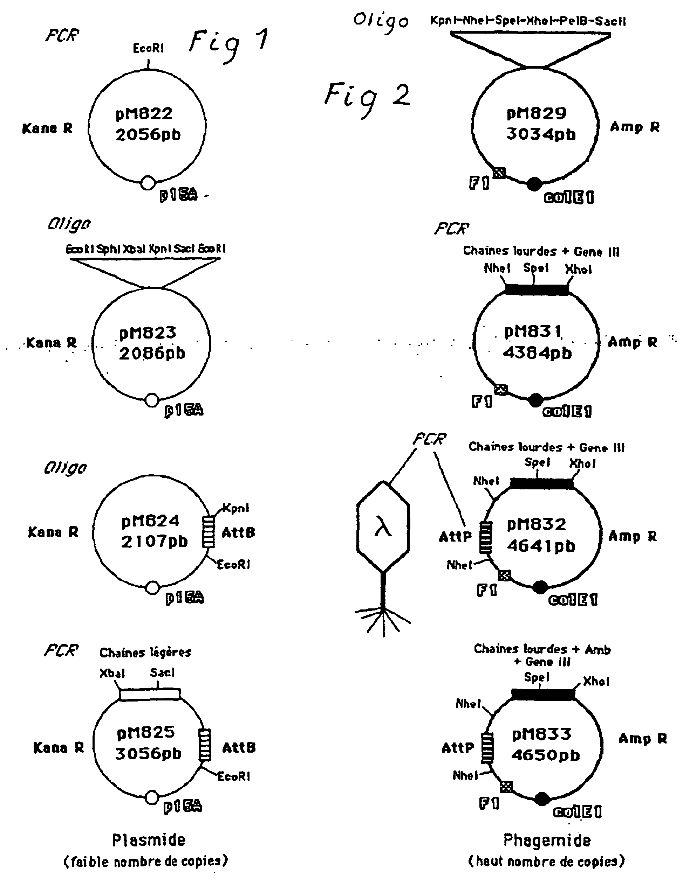

La figure 1 représente schématiquement les étapes de constructions partant de pM822 pour aboutir à pM825.
La figure 2 représente schématiquement les étapes de construction partant de pM829 pour aboutir à pM833.
La figure 3 représente schématiquement l'étape de recombinaison de pM827 et pM834.
La figure 4 représente une vue schématique du vecteur multicombinatoire pM835 obtenu.
La figure 5 représente les amorces d'amplification du pACYC177.
La figure 6 représente la séquence du linker 30 pb.
La figure 7 représente l'oligonucléotide incorporant la séquence de recombinaison AttB.
La figure 8 représente la séquence du linker 70 pb.
La figure 9 représente les amorces d'amplification du promoteur lac.
La figure 10 représente la séquence du linker 98 pb.
La figure 11 représente les amorces d'amplification de la chaîne V_{L} anti-gp 160.
La figure 12 représente les amorces d'amplification de pM825.
La figure 13 représente les amorces d'amplification du gène de résistance au chloramphénicol.
La figure 14 représente la séquence du linker 68 pb.
La figure 15 représente la séquence du linker 115 pb.
La figure 16 représente les amorces d'amplification du gène III.
La figure 17 représente les amorces d'amplification de la chaîne V_{H} anti-gp 160.
La figure 18 représente les amorces d'amplification de la séquence de recombinaison AttP.
La figure 19 représente les amorces d'amplification de la chaîne lourde introduisant la mutation Ambre.
La figure 20 représente les amorces d'amplification du promoteur du gène de résistance au chloramphénicol.

### I - Création d'un plasmide possédant une origine de réplication p15A, un gène de résistance à la kanamycine et une séquence de recombinaison AttB pour l'insertion du gène ou de banque de gènes régions variables de chaîne légère.

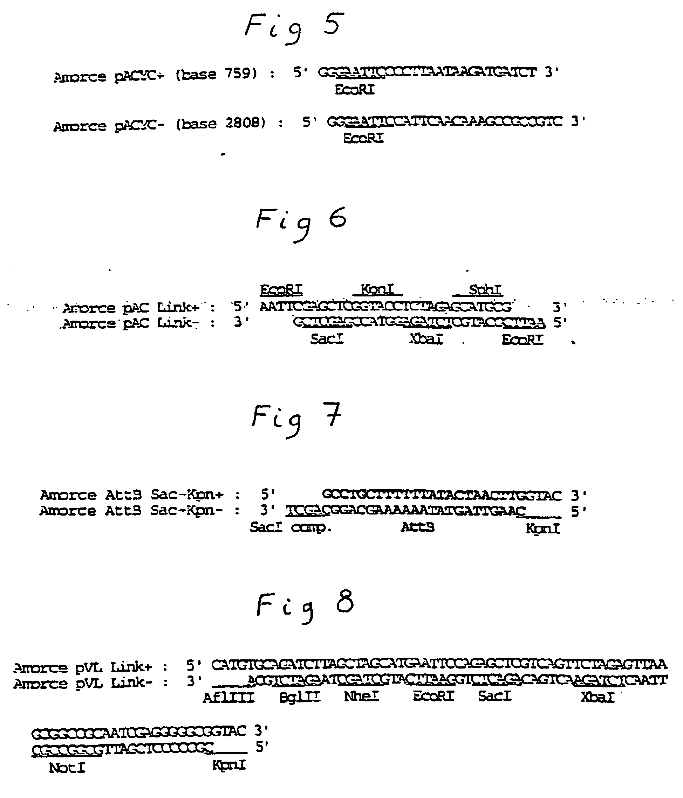

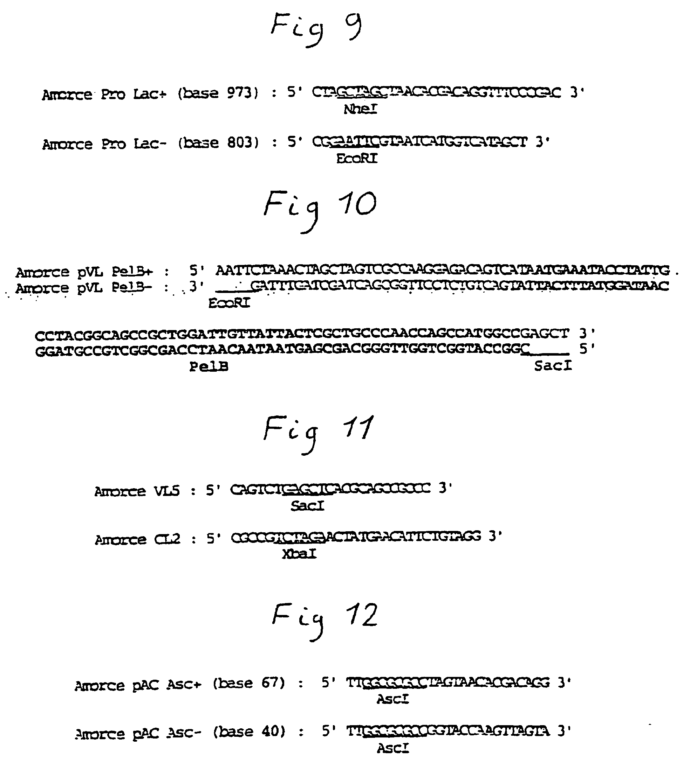

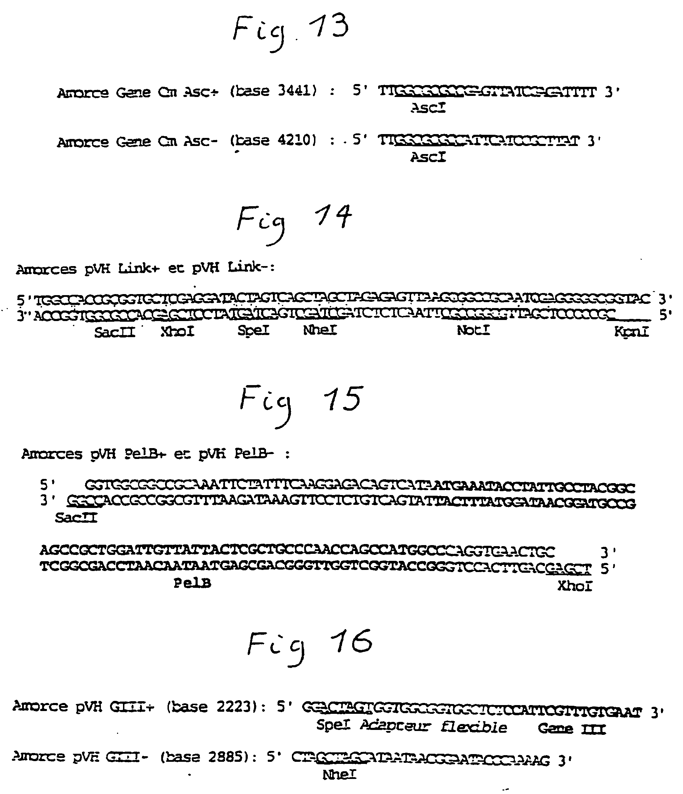

1) On procède à l'amplification par PCR (réaction en chaîne de polymérase) d'un fragment de 2 050 pb couvrant les bases 759 à. 2 810 du plasmide pACYC177. Ce plasmide (ATCC 37031), diffusé par la firme Biolabs, est répertorié, et sa séquence décrite, dans les banques de données (GenBank Accession n° X06402). Ce fragment amplifié comprend le gène de résistance à la kanamycine, y compris son promoteur, l'origine de réplication p15A et un site EcoRI à chaque extrémité. Le fragment amplifié par PCR est ensuite recircularisé pour former un plasmide appelé pM822, de 2 056 pb.
   Les amorces utilisées pour l'amplification du fragment sont décrites sur la figure 5 et dans les identificateurs SEQ ID No. 1 et 2.
2) Dans le site EcoRI unique du vecteur pH822 on insère un adapteur (linker) synthétique de 30 pb comprenant les sites de restriction SacI, KpnI, XbaI et SphI, le vecteur obtenu, de 2 086 pb étant appelé pM823. Les séquences d'amorce adapteur (linker) synthétique apparaissent sur la figure 6 et dans les identificateurs SEQ ID No. 3 et 4.
3) On insère dans le vecteur pM823 la séquence de recombinaison AttB de 23 pb sous forme d'oligonucléotide synthétique entre les sites SacI et KpnI, en contrôlant l'orientation 5'-3' par destruction du site SacI par modification de sa dernière base. On obtient ainsi un plasmide pM824 de 2 107 pb. Les séquences oligonucléotidiques synthétiques correspondant au site de recombinaison AttB sont décrites sur la figure 7 et les identificateurs SEQ ID No. 5 et 6.
4) Insertion de la cassette permettant le clonage des parties variables des chaînes légères V_{L}.
   - pour construire un phagemide appelé pM452 on procède à la digestion du phagemide pBLuescript IISK+ (commercialisé par Stratagène) de 2 961 pb par les enzymes AF1III et KpnI et à l'élimination d'un fragment de 501 pb comprenant le promoteur lactose et différents sites de clonage. On synthétise un adapteur de 70 pb représenté sur la figure 8, dont les séquences d'amorces sont décrites dans les identificateurs SEQ ID No. 7 et 8 comprenant les sites de restriction BglII, NheI, EcoRI, SacI, XbaI et NotI, et on insère le linker entre AflIII et KpnI pour aboutir à un phagemide pM836 de 2 530 pb.
   - On insère entre les sites NheI et EcoRI un fragment de 171 pb amplifié par PCR à partir du pBluescript (bases 802 à 973) en utilisant les amorces correspondant aux identificateurs de séquence SEQ ID No. 9 et 10 (figure 9), ce fragment contenant le promoteur lac pour obtenir le phagemide pM837.
   - On insère entre EcoRI et SacI de pM837, un adapteur synthétique de 98 pb contenant la séquence signal PelB (figure 10 et identificateurs SEQ ID No. 11 et 12) pour obtenir finalement un phagemide pH838 de 2 795 pb.
   - On insère ensuite la séquence codant pour la région V_{L} de chaîne légère d'un clone anti-gp 160 de HIV de 642 pb amplifiée par PCR (banque d'ADNc) entre les sites SacI et XbaI, ce qui permet la conservation de la phase de lecture entre PelB et la chaîne légère. Les amorces (figure 11) sont définies dans les identificateurs de séquence SEQ ID No. 13 et 14. On obtient le phagemide pM452 (3 427 pb) contenant la cassette V_{L} (promoteur lac, séquence signal PelB et la séquence (642 pb) codant pour la chaîne légère du clone anti-gp 160.
   - Le phagemide pM452 est digéré par NheI et XbaI pour libérer un fragment de 960 pb correspondant à la cassette V_{L}, fragment que l'on purifie.
   - Ce fragment est ensuite inséré dans le vecteur pM824 au niveau des sites KpnI et XbaI avec destruction du site KpnI pour contrôler l'orientation, donnant le plasmide pM825 de 3 056 pb.
5) Un site AscI unique est créé en position 3' de la séquence AttB par amplification par PCR du plasmide pM825 entier à l'aide de deux amorces (figure 12) possédant un site AscI à leur extrémité et ayant les séquences figurant sur les identificateurs SEQ ID No. 15 et 16. Le plasmide ainsi modifié est nommé plasmide pM826 (3 050 pb).

Le gène de résistance au chloramphénicol (770 pb), dépourvu de son promoteur, est amplifié par PCR à partir du plasmide pBR328 (Boehringer, Accession Genbank VB0004). L'amplification est faite avec les amorces (figure 13) dont les séquences sont indiquées sur les identificateurs de séquences SEQ ID No. 17 et 18. On clone ensuite le gène de résistance au chloramphénicol dans le plasmide pM826 au niveau du site AscI en déterminant l'orientation correcte pour son expression après l'étape de recombinaison, par séquençage. On obtient ainsi un plasmide dénommé pM827 de 3 816 pb.

### II - Création d'un vecteur de type phagemide portant deux origines de réplication ColE1 et f1, un gène de résistance à l'ampicilline et une séquence de recombinaison AttP pour l'insertion d'une banque de régions variables de chaînes lourdes.

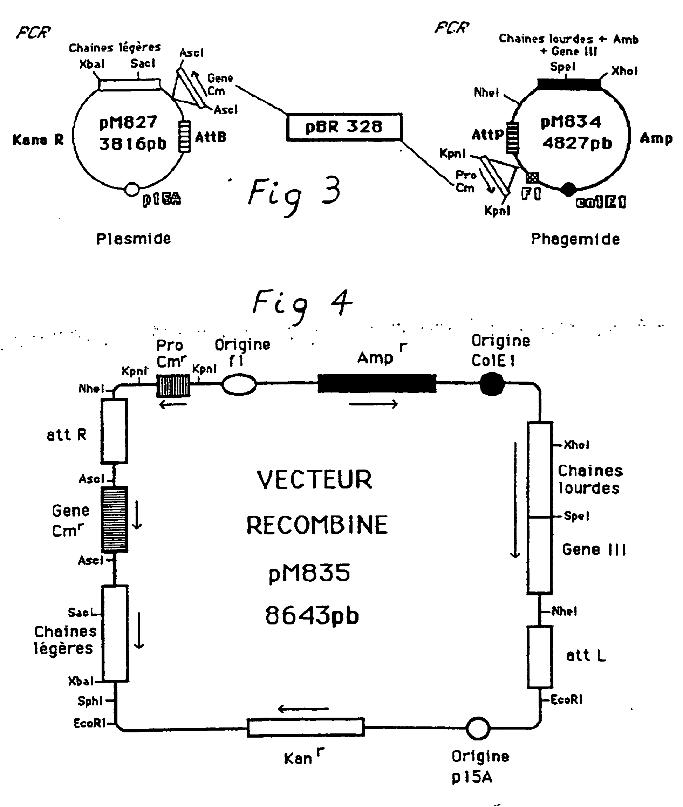

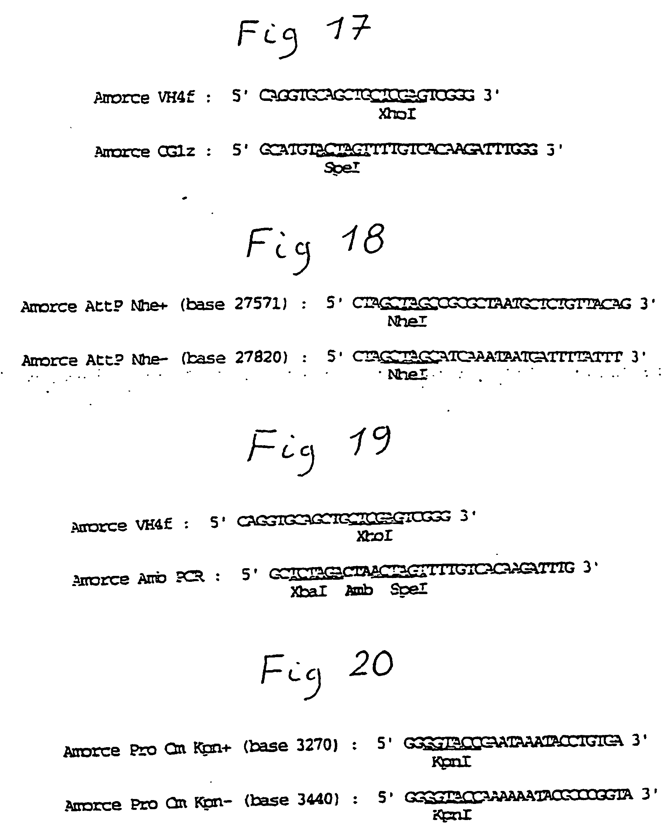

1) Modification du vecteur pBluescript SKII+ (Stratagène, Accession Genbank X52328) pour obtenir un phagemide capable de présenter un Fab fusionné au niveau du gène III (protéine de surface) à sa surface :
   - Après digestion du pBluescript par SacI et destruction du site SacI par action de la mung bean nucléase puis digestion par KpnI pour éliminer le site de clonage multiple (polylinker) (107 pb), on insère un adapteur synthétique de 68 pb (figure 14) contenant les sites SacII, XhoI, SpeI, NheI et NotI (séquences SÉQ ID n° 19 et 20), obtenant le phagemide pMB28 de 2 922 pb.
   - On crée un adapteur synthétique 115 pb (SEQ ID No. 21 et 22) contenant la séquence signal PelB. Ce linker (figure 15) est inséré entre les sites SacII et XhoI pour donner le phagemide pM829 de 3 034 pb.
   - On procède à l'insertion, entre les sites SpeI et Nhel de pM828 d'un adapteur flexible de 15 pb (fig 16 et identificateurs SEQ ID No. 23 et 24) et du gène III du phage M13 (663 pb) après amplification par PCR sur le phage M13 mp18 des bases 2 223 à 2 885 (Biolabs, Accession Genbank X02513), obtenant le phagemide pM830 de 3 709 pb.
   - On amplifie par PCR la chaîne lourde d'un clone (ADNc) anti-gp 160 de HIV de 684 pb à l'aide des amorces SEQ ID No. 25, 26 (figure 17). Cette chaîne lourde est ensuite insérée entre les sites XhoI et SpeI du phagemide pM830 pour aboutir au phagemide pM831 de 4 384 pb, avec conservation de la phase de lecture entre PelB et la chaîne lourde et entre celle-ci et le gène III.
2) Insertion de la séquence de recombinaison AttP de 250 pb au niveau du site NheI après amplification par PCR sur le phage λ des bases 27 571 à 27 820 (Biolabs, Accession Genbank V00636). Les amorces utilisées (figure 18) possèdent les séquences SEQ ID No. 27 et 28. La séquence de recombinaison AttP amplifiée peut être insérée avec deux orientations possibles mais seule une orientation est retenue.On aboutit ainsi au phagemide pM832 de 4 641 pb comprenant la structure représentée sur la figure 3.
3) Modification du vecteur pour la production de Fab solubles.
   Pour produire facilement des Fab solubles, une mutation Ambre (identifiée comme un codon stop dans les souches bactériennes non suppressives) est donc introduite entre la chaîne lourde et le gène III en conservant la phase. La chaîne lourde est amplifiée par PCR avec une amorce en 5' possédant le site XhoI (SEQ ID No. 25) et une autre en 3' (SEQ ID No. 29 ; voir figure 19) contenant le site SpeI suivi du codon Ambre TAG puis du site Xbal compatible avec SpeI. Elle est ensuite clonée entre les sites SpeI et XhoI du vecteur pM832, aboutissant au phagemide pM833 de 4 650 pb.
4) Insertion du promoteur du gène de résistance au chloramphénicol au niveau du site unique KpnI situé en 3' de la séquence de recombinaison AttP. Une seule orientation confère une nouvelle résistance au vecteur recombiné. Le promoteur (177 pb) est amplifié préalablement par PCR à partir des bases 3270 à 3440 du vecteur pBR328 (commercialisé par Biolabs) à l'aide des amorces (figure 20) dont les séquences sont indiquées dans les identificateurs de séquence SEQ ID No. 30 et 31. On obtient ainsi le phagemide pM834 de 4 827 pb.

### III Recombinaison entre les deux vecteurs pM827 et pM834.

Les deux plasmides précités serviront de point de départ pour obtenir des banques d'anticorps multicombinatoires. Dans l'exemple représenté la recombinaison s'effectuera entre les chaînes V_{L} et V_{H} du clones anti-gp 160 de HIV utilisé. Cependant si les deux vecteurs sont construits à partir de banques de gènes de chaînes lourdes et/ou de gènes de chaînes légères d'anticorps, ils permettront d'obtenir une banque multicombinatoire d'anticorps que l'on pourra ensuite cribler.

Transformés dans une souche adéquate (D1210HP), les deux vecteurs pourront s'associer grâce aux séquences AttP et AttB, cibles d'un facteur de recombinaison int inductible. Les mécanismes de recombinaison sont décrits dans le chapitre 9 du livre "The recombination of genetic material" (Miller H. V., Viral and cellular control of site-specific recombination, 1988, p 360-384, edited by Brooks Low K., Academic Press). Le vecteur multicombinatoire (8 643 pb) ainsi créé possèdera trois origines de réplication différentes, trois résistances aux antibiotiques et les deux cassettes permettant l'expression des chaînes VL et VH.
1) On procède à la transformation par électroporation de la souche de E. coli D1210HP (diffusée par Stratagène), et qui contient la recombinase lnductible int, avec l'épisome F' sous forme d'ADN provenant de la souche bactérienne XL1-Blue (diffusée par Stratagène). Les bactéries sont ainsi Infectables par un phage filamenteux. L'épisome F' est maintenu dans la souche grâce à sa résistance à la tétracycline. La souche D1210HP-F' ainsi transformée possède le génotype suivant : D1210HP-F' : HB101, lacI^{q}, lacY⁺, λxis⁻ kil⁻ cI857 [F', proAB, lacI^{q}ZAM15, Tn10(Tet^{R})].
2) Etapes de la recombinaison.
   - On procède à la transformation de cette souche par le plasmide pM 827 (contenant les chaînes V_{L} et résistant à la kanamycine), et en parallèle, on transforme une souche compatible, par exemple, XL-1 Blue par le phagemide pM834 (résistant à l'ampicilline et portant les chaînes VH).

A partir de la souche transformée par le phagemide pM 834, on prépare celui-ci sous forme de phage puis on Infecte la culture de D1210 HP-F' contenant le plasmide pM 827, à 30°C (DO = 0,6).

Après 30 mn d'infection à 30°C, on soumet la culture à un choc thermique à 42°C pendant une heure pour déclencher la recombinaison sous l'effet de la recombinase inductible.

Après avoir remis la culture à 30°C et ajouté du chloramphénicol (50 à 100 µg/ml final), on étale les clones sur milieu gélosé contenant du chloramphénicol. On ajoute une heure après le phage helper VCSM13 (Kan^{R}) de Stratagène à raison de 10¹² pfu/100 ml de culture. Puis on ajoute, deux heures après, la kananycine à 70 µg/ml final et la culture est laissée quelques heures à 30°C.

L'analyse des clones après recombinaison des vecteurs pM827 et pM834 montre que l'association s'est bien déroulée. On obtient un phagemide recombiné pM835 de 8 643 pb stable, exprimant un Fab et toujours capable d'infecter la souche D1210HP-F. Les points de jonction AttL et AttR ont été vérifiés par séquençage.

### IV - Préparation d'une banque multicombinatoire à partir d'une banque de chaînes légères et d'une banque de chaînes lourdes.

L'étape d'insertion I.4 de la région variable de la chaîne légère du clone anti-gp 160 de HIV amplifié par PCR est remplacé par une insertion similaire des régions variables des chaînes légères d'une banque de chaînes légères d'anticorps amplifiée par PCR à l'aide d'un système d'amorces connu convenable propre au type de chaîne légère recherchée, ou d'une pluralité de systèmes d'amorces si l'on souhaite effectuer la multicombinaison à partir de populations de différents types de chaînes légères. Les systèmes d'amorce permettant l'amplification des chaînes légères sont bien connus et décrits par W.D. Huse et al., Science, Vol 246 (1989), 1275-1281.

De la même façon, pour le clonage des chaînes lourdes, on remplace l'étape d'insertion II.1 de la chaîne lourde d'un clone anti-gp 160 HIV par l'insertion d'une banque de régions variables de chaînes lourdes amplifiée par PCR à l'aide des systèmes d'amorce convenables décrits par M.J. Campbell et al., Molecular Immunology, Vol. 29, No. 2 (1992), 193-203 ou par W.D. Huse et al. ci-dessus.

Après les étapes de recombinaison les clones résistant au chloramphénicol sont sélectionnés et on procède ensuite au criblage destiné à sélectionner les clones exprimant des associations de chaîne légère et chaîne lourde d'anticorps ayant les affinités recherchées contre les antigènes déterminés, conformément aux techniques décrites dans par exemple par S.F. Parmley et al., Gene 73 (1988), 305-318.

## Revendications

1. Procédé de production de banques multicombinatoires dans lequel, partant d'un premier répertoire de gènes codant pour une population d'un de deux types de polypeptides susceptibles de s'associer, notamment de régions variables de l'un des types chaîne légère et chaîne lourde d'anticorps, et d'au moins d'un gène codant pour l'autre type de polypeptide, notamment une région variable de l'autre type de chaîne d'anticorps, ou de préférence d'un second répertoire de gènes codants pour une population dudit autre type, on introduit les gènes dudit premier répertoire dans un premier vecteur pour former une population de vecteurs portant les différents gènes dudit premier répertoire, et on introduit ledit gène dudit autre type ou les gènes dudit second répertoire dans un second vecteur, l'un au moins desdits vecteurs de type phagemide, dit receveur, étant agencé pour recevoir, par recombinaison avec l'autre vecteur de type plasmide, tout ou partie dudit autre vecteur, avec son gène, dans des conditions permettant audit vecteurs phagemide receveur de contenir, après recombinaison, un gène de l'un des deux types et un gène de l'autre type et d'exprimer les deux gènes sous forme de polypeptides associés susceptibles d'apparaître à la surface extérieure d'un phage en y restant maintenus et en étant associée entre eux de façon multimérique ou simulant un multimère,
**caracterisé en ce que** lesdits premier et second vecteurs contiennent respectivement l'un des sites de recombinaison spécifique attB de E. coli et attP du phage λ, agencé de façon à permettre la recombinaison sous l'effet d'une recombinase ou Intégraàe associée en formant de façon irréversible, dans un vecteur unique résultant de la recombinaison, des séquences de jonction stables attL et attR.

2. Procédé selon la revendication 1, **caractérisé en ce que** le vecteur recombinant obtenu à partir desdits premier et second vecteurs est agencé pour présenter un marqueur de sélection initialement non fonctionnel et rendu fonctionnel par la recombinaison.

3. Procédé selon la revendication 2, **caractérisé en ce que** le marqueur de sélection comporte un gène permettant la sélection, lorsqu'il est exprimé, et un promoteur propre au gène, le promoteur étant inséré dans l'un desdits premier et second vecteurs et le gène marqueur dans l'autre.

4. Procédé selon la revendication 3, **caractérisé en ce que** le marqueur est le gène de résistance à un antibiotique, notamment un gène du groupe formé par les gènes de résistance aux tétracyclines, à la gentamycine et au chloramphénicol, avec son promoteur.

5. Procédé selon l'une des revendications 1 à 4, **caractérisé en ce que** l'on utilise, pour contrôler l'étape de recombinaison entre le premier et le second vecteur, une recombinase thermo-inductible.

6. Vecteurs phagemides susceptibles d'être obtenus par le procédé selon l'une des revendications 3 à 5, et qui se caractérisent par la présence d'une séquence codant pour l'un de deux types de polypeptides susceptibles de s'associer, notamment une partie variable de chaîne légère d'anticorps, et une séquence codant pour l'autre desdits deux types, notamment une partie variable de chaîne lourde d'anticorps, lesdites séquences étant accompagnées, dans des cadres convenables, des éléments permettant leur expression dans un hôte, lesdites séquences de chaîne légère et de chaîne lourde, avec lesdits moyens qui leur sont associés, étant respectivement séparées par des séquences relatives à des sites uniques d'intégration attR ou attL.

7. Banques multicombinatoires de vecteurs, formées par les vecteurs selon la revendication 6 et associant, de façon aléatoire, une séquence codant pour l'un de deux types de polypeptides susceptibles de s'associer, notamment une partie variable de chaîne légère d'anticorps et une séquence codant pour l'autre desdits deux types, notamment une partie variable de chaîne lourde d'anticorps.

## Patentansprüche

1. Verfahren zur Herstellung von multikombinatorischen Banken (Bibliotheken), bei dem man ausgehend von einem ersten Repertoir von Genen, die für eine Population von einem von zwei Arten von Polypeptiden kodieren, welche sich assoziieren können, insbesondere mit variablen Regionen von einem der Typen von leichter Kette und schwerer Kette von Antikörpern, und mindestens einem Gen, das für die andere Art von Polypeptid kodiert, insbesondere eine variable Region des anderen Typs von Antikörper-Kette, oder vorzugsweise einem zweiten Repertoir von Genen, die für eine Population der anderen Art kodieren,
die Gene des ersten Repertoirs in einen ersten Vektor insertiert zur Bildung einer Population von Vektoren, welche die verschiedenen Gene des ersten Repertoirs tragen, und
das Gen der anderen Art oder die Gene des zweiten Repertoirs in einen zweiten Vektor insertiert,
wobei mindestens einer der Vektoren des Phagemid-Typs, der als Empfänger bezeichnet wird, dazu in der Lage ist, durch Rekombination mit dem anderen Vektor des Plasmid-Typs den gesamten oder einen Teil des anderen Vektors mit seinem Gen bei Bedingungen aufzunehmen, die es dem genannten Phagemid-Vektor ermöglichen, nach der Rekombination ein Gen der einen der beiden Arten und ein Gen der anderen Art zu enthalten und die beiden Gene in Form von assoziierten Polypeptiden zu exprimieren, welche dazu in der Lage sind auf der äußeren Oberfläche eines Phagen zu erscheinen und dort zu verbleiben, indem sie miteinander nach Art eines Multimers assoziiert sind oder ein Multimer simulieren,
**dadurch gekennzeichnet, daß** die ersten und zweiten Vektoren eine der spezifischen Rekombinationsstellen attB von E. coli beziehungsweise attP des Phagen λ enthalten, welche die Rekombination unter der Einwirkung einer assoziierten Rekombinase oder Integrase ermöglicht, indem in irreversibler Weise in einem einzigen bei der Rekombination gebildeten Vektor stabile Verknüpfungssequenzen attL und attR gebildet werden.

2. Verfahren nach Anspruch 1, **dadurch gekennzeichnet, daß** der ausgehend von den ersten und zweiten Vektoren erhaltene rekombinante Vektor dazu in der Lage ist, einen Selektionsvektor zu zeigen, der anfänglich nichtfunktionell ist und durch die Rekombination funktionell gemacht wird.

3. Verfahren nach Anspruch 2, **dadurch gekennzeichnet, daß** der Selektionsmarker ein Gen, welches nach der Exprimierung die Selektion ermöglicht und einen dem Gen eigenen Promotor aufweist, wobei der Promotor in einen der ersten und zweiten Vektoren und das Markergen in den anderen insertiert werden.

4. Verfahren nach Anspruch 3, **dadurch gekennzeichnet, daß** der Marker das Resistenzgen für ein Antibiotikum, insbesondere ein Gen der Gruppe, welches durch Gene für die Resistenz gegen Tetracycline, Gentamicin und Chloramphenicol gebildet ist, zusammen mit seinem Promotor ist.

5. Verfahren nach einem der Ansprüche 1 bis 4, **dadurch gekennzeichnet, daß** man zur Steuerung der Stufe der Rekombination zwischen dem ersten und dem zweiten Vektor eine thermo-induzierbare Rekombinase verwendet.

6. Phagemid-Vektoren erhältlich nach dem Verfahren gemäß einem der Ansprüche 3 bis 5, **gekennzeichnet durch** die Anwesenheit einer Sequenz, die für eine von zwei Arten von Polypeptiden kodiert, welche sich assoziieren können, insbesondere mit einem variablen Teil der leichten Antikörper-Kette, und einer Sequenz, die für die andere der beiden Arten von Polypeptiden kodiert, welche Sequenzen in geeigenten Rahmen von Elementen begleitet sind, die ihre Expression in einem Wirt ermöglichen, wobei die genannten Sequenzen der leichten Kette und der schweren Kette zusammen mit den mit ihnen assoziierten Mitteln **durch** Sequenzen relativ zu den einzigen Integrationsstellen attR beziehungsweise attL getrennt sind.

7. Multikombinatorische Vektoren-Banken (-Bibliotheken), gebildet durch die Vektoren gemäß Anspruch 6 und in Assoziation in aleatorischer Weise mit einer Sequenz, die für eine von zwei Arten von Polypeptiden kodiert, welche sich assoziieren können, insbesondere mit einem variablen Teil der leichten Antikörper-Kette, und einer Sequenz, die für die andere der beiden Arten von Polypeptiden kodiert, insbesondere einem variablen Teil der schweren Antikörper-Kette.

## Claims

1. Method for preparing multicombinatorial libraries wherein, starting from a first repertoire of genes coding for a population of one of two kinds of polypeptides capable of being combined, particularly either an antibody light chain variable region or an antibody heavy chain variable region, and at least one gene coding for the other type of polypeptide, notably a variable region of the other type of antibody chain, or preferably a second repertoire of genes coding for a population of said other type, the genes of said first repertoire are inserted in a first vector to form a vector population carrying the different genes of said first repertoire, and said gene of said second type or the genes of said second repertoire are introduced into a second vector, at least one of said vectors of the phagemide type, known as the receiver, being adapted to receive, by recombination with the other vector of the plasmid type, all or part of said other vector, with its gene, under conditions enabling said phagemide receiving vector to contain, after recombination, a gene of one of the two types and a gene of the other type and to express the two genes in the form of combined polypeptides capable of appearing on the outer surface of a phage while remaining held thereon and being associated with one another in multimeric manner or in a manner resembling a multimer, **characterised in that** the first and second vectors respectively contain one of the specific attB recombination sites for E. coli and attP recombination sites for the lambda phage, arranged so as to allow recombination under the effect of an associated recombinase or integrase while irreversibly forming, in a single vector produced by the recombination, stable attL and attR junction sequences.

2. Method according to claim 1, **characterised in that** the recombinant vector obtained from said first and second vectors is arranged to have a selectable marker which is initially non-functional and is rendered functional by the recombination.

3. Method according to claim 2, **characterised in that** the selectable marker comprises a gene which allows selection, when it is expressed, and a promoter belonging to the gene, the promoter being inserted in one of said first and second vectors and the marker gene being inserted in the other.

4. Method according to claim 3, **characterised in that** the marker is the resistance gene to an antibiotic, notably a gene of the group formed by the resistance genes to tetracyclines, gentamycine and chloramphenicol, with the promoter thereof.

5. Method according to one of claims 1 to 4, **characterised in that** a heat-inducible recombinase is used to control the recombination step between the first and second vectors.

6. Phagemide vectors which may be obtained by the method according to one of claims 3 to 5, which are **characterised by** the presence of a sequence coding for one of two types of polypeptides capable of being combined, notably an antibody light chain variable region, and a sequence coding for the other of the two types, notably an antibody heavy chain variable region, said sequences being accompanied, within suitable frames, by elements enabling them to be expressed in a host, said light and heavy chain sequences, with said means associated with them, being respectively separated by sequences relating to single attR or attL integration sites.

7. Multicombinatorial vector libraries, formed by the vectors according to claim 6 and randomly combining a sequence coding for one of two types of polypeptides capable of being combined, notably an antibody light chain variable region and a sequence coding for the other of the two types, notably an antibody heavy chain variable region.


### LISTE DE SEQUENCES

(1) INFORMATION GENERALE:
   (i) DEPOSANT:
      (A) NOM: PASTEUR MERIEUX Sérums et Vaccins
      (B) RUE: 58 avenue Leclerc
      (C) VILLE: LYON
      (E) PAYS: FRANCE
      (F) CODE POSTAL: 69007
   (ii) TITRE DE L' INVENTION: Procédé de préparation d'une banque multicombinatoire de vecteurs d'expression de gènes d'anticorps, banque et systèmes d'expression d'anticorps "colicionaux" obtenus.
   (iii) NOMBRE DE SEQUENCES: 31
   (iv) FORME LISIBLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patent In Release #1.0, Version #1.25 (OEB)
(2) INFORMATION POUR LA SEQ ID NO: 1:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce pACYC+
   (xi) DESCRIPTION DE LA SEQUENCE: SEQ ID NO: 1:
   GGGAATTCCC CTTAATAAGA TGATCT 26
(2) INFORMATION POUR LA SEQ ID NO: 2:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce pACYC-
   (xi) DESCRIPTION DE LA SEQUENCE: SEQ ID NO: 2:
   GGGAATTCCA TTCAACAAAG CCGCCGTC 28
(2) INFORMATION POUR LA SEQ ID NO: 3:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse amorce linker pAC Llnk+
   (xi) DESCRIPTION DE LA SEQUENCE: SEQ ID NO: 3:
   AATTCGAGCT CGGTACCTCT AGAGCATGCG 30
(2) INFORMATION POUR LA SEQ ID NO: 4 :
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pAC Link-
   (xi) DESCRIPTION DE LA SEQUENCE: SEQ ID NOs 4:
   AATTCGCATG CTCTAGAGGT ACCGAGCTCG 30
(2) INFORMATION POUR LA SEQ ID NO: 5:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : séquence de recombinaison AttB Sac-Kpn+
   (xi) DESCRIPTION DE LA SEQUENCE: SEQ ID NO: 5:
   GCCTGCTTTT TTATACTAAC TTGGTAC 27
(2) INFORMATION POUR LA SEQ ID NO, 6:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse : séquence de recombinaison AttB Sac-Kpn-
   (xi) DESCRIPTION DE LA SEQUENCE: SEQ ID NO: 6:
(2) INFORMATION POUR LA SEQ ID NO: 7:
   (i) CARACTERISTIQUES DE LA SEQUENCE:
      (A) LONGUEUR: 79 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pVL Link+
   (xi) DESCRIPTION DE LA SEQUENCE: SEQ ID NO: 7:
   CATGTGCAGA TCTTAGCTAG CATGAATTCC AGAGCTCGTC AGTTCTAGAG TTAAGCGGCC 60
   GCAATCGAGG GGGCGGTAC 79
(2) INFORMATION POUR LA SEQ ID NO: 8:
   (i) CARACTERISTIQUES DE LA SEQUENCE:
      (A) LONGUEUR: 71 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pVL Link-
   (xi) DESCRIPTION DE LA SEQUENCE: SEQ ID NO: 8:
   CGCCCCCTCG ATTGCGGCCG CTTAACTCTA GAACTGACAG ACTCTGGAAT TCATGCTAGC 60
   TAAGATCTGC A 71
(2) INFORMATION POUR LA SEQ ID NO: 9:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Pro Lac+
   (xi) DESCRIPTION DE LA SEQUENCE: SEQ ID NO: 9:
   CTAGCTAGCT AACACGACAG GTTTCCCGAC 30
(2) INFORMATION POUR LA SEQ ID NO: 10:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Pro Lac-
   (xi) DESCRIPTION DE LA SEQUENCE: SEQ ID NO: 10:
   CGGAATTCGT AATCATGGTC ATAGCT 26
(2) INFORMATION POUR LA SEQ ID NO: 11:
   (i) CARACTERISTIQUES DE LA SEQUENCE.
      (A) LONGUEUR: 108 paires de bases
      (B) TYPE: acide nucléique
      (C) NOMBRE DE BRINS: simple
      (D) CONFIGURATION: linéaire
   (ii) TYPE DE MOLECULE : Ollgonucléotide de synthèse amorce linker pVL PelB+
   (xi) DESCRIPTION DE LA SEQUENCE: SEQ ID NO: 11:
   AATTCTAAAC TAGCTAGTCG CCAAGGAGAC AGTCATAATG AAATACCTAT TGCCTACGGC 60
   AGCCGCTGGA TTGTTATTAC TCGCTGCCCA ACCAGCCATG GCCGAGCT 108
(2) INFORMATION POUR LA SEQ ID NO: 12.
   (i) CARACTERISTIQUES DE LA SEQUENCE:
      (A) LONGUEUR: 100 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse : amorce linker pVL PelB-
   (xi) DESCRIPTION DE LA SEQUENCE: SEQ ID NO: 12:
   CGGCCATGGC TGGTTGGGCA GCGAGTAATA ACAATCCAGC GGCTGCCGTA GGCAATAGGT 60
   ATTTCATTAT GACTGTCTCC TTGGCGACTA GCTAGTTTAG 100
(2) INFORMATION POUR LA SEQ ID NO: 13:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse : amorce VL5
   (xi) DESCRIPTION DE LA SEQUENCE: SEQ ID NO: 13:
   CAGTCTGAGC TCACGCAGCC GCCC 24
(2) INFORMATION POUR LA SEQ ID NO: 14:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce CL2
   (xi) DESCRIPTION DE LA SEQUENCE: SEQ ID NO: 14:
   CGCCGTCTAG AACTATGAAC ATTCTGTAGG 30
(2) INFORMATION POUR LA SEQ ID NO: 15:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse : amorce pAC Asc+
   (xi) DESCRIPTION DE LA SEQUENCE: SEQ ID NO: 15:
   TTGGCGCGCC TAGTAACACG ACAGG 25
(2) INFORMATION POUR LA SEQ ID NO: 16:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce pAC Asc-
   (xi) DESCRIPTION DE LA SEQUENCE: SEQ ID NO: 16:
   TTGGCGCGCC GGTACCAAGT TAGTA 25
(2) INFORMATION POUR LA SEQ ID NO: 17:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Gene Cm Asc+
   (xi) DESCRIPTION DE LA SEQUENCE: SEQ ID NO: 17:
   TTGGCGCGCC GAGTTATCGA GATTTT 26
(2) INFORMATION POUR LA SEQ ID NO: 18:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE : oligonucléotide de synthèse : amorce Gène Cm Asc-
   (xi) DESCRIPTION DE LA SEQUENCE: SEQ ID NO: 18:
   TTGGCGCGCC ATTCATCCGC TTAT 24
(2) INFORMATION POUR LA SEQ ID NO: 19:
   (i) CARACTERISTIQUES DE LA SEQUENCE:
      (A) LONGUEUR: 73 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pvH Link+
   (xi) DESCRIPTION DE LA SEQUENCE: SEQ ID NO: 19:
(2) INFORMATION POUR LA SEQ ID NO: 20:
   (i) CARACTERISTIQUES DE LA SEQUENCE:
      (A) LONGUEUR: 69 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pVH Link-
   (xi) DESCRIPTION DE LA SEQUENCE: SEQ ID NO: 20:
   CGCCCCCTCG ATTGCGGCCG CTTAACTCTC TAGCTAGCTG ACTAGTATCC TCGAGCACCG 60
   CGGTGGCCA 69
(2) INFORMATION POUR LA SEQ ID NO: 21:
   (i) CARACTERISTIQUES DE LA SEQUENCE:
      (A) LONGUEUR: 117 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pVH PelB+
   (xi) DESCRIPTION DE LA SEQUENCE: SEQ ID NO: 21:
   GGTGGCGGCC GCAAATTCTA TTTCAAGGAG ACAGTCATAA TGAAATACCT ATTGCCTACG 60
   GCAGCCGCTG GATTGTTATT ACTCGCTGCC CAACCAGCCA TGGCCCAGGT GAACTGC 117
(2) INFORMATION POUR LA SEQ ID NO: 22:
   (i) CARACTERISTIQUES DE LA SEQUENCE:
      (A) LONGUEUR: 123 paires de bases
      (B) TYPE: acide nucléique
      (C) NOMBRE DE BRINS: simple
      (D) CONFIGURATION: linéaire
   (ii) TYPE DE MOLECULE :Oligonucléotide de synthèse : amorce linker pVH PelB-
   (xi) DESCRIPTION DE LA SEQUENCE: SEQ ID NO: 22:
   TCGAGCAGTT CACCTGGGCC ATGGCTGGTT GGGCAGCGAG TAATAACAAT CCAGCGGCTG 60
   CCGTAGGCAA TAGGTATTTC ATTATGACTG TCTCCTTGAA ATAGAATTTG CGGCCGCCAC 120
   CGG 123
(2) INFORMATION POUR LA SEQ ID NO: 23:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse amorce linker pVH GIII+
   (xi) DESCRIPTION DE LA SEQUENCE: SEQ ID NO: 23:
   GGACTAGTGG TGGCGGTGGC TCTCCATTCG TTTGTGAAT 39
(2) INFORMATION POUR LA SEQ ID NO: 24:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker pVH GIII-
   (xi) DESCRIPTION DE LA SEQUENCE: SEQ ID NO: 24:
   CTAGCTAGCA TAATAACGGA ATACCCAAAA G 31
(2) INFORMATION POUR LA SEQ ID NO: 25:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE : Oliconucléotide de synthèse : amorce linker VH4f
   (xi) DESCRIPTION DE LA SEQUENCE: SEQ ID NO: 25:
   CAGGTGCAGC TGCTCGAGTC GGG 23
(2) INFORMATION POUR LA SEQ ID NO: 26:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce linker CGlz
   (xi) DESCRIPTION DE LA SEQUENCE: SEQ ID NO: 26:
   GCATGTACTA GTTTTGTCAC AAGATTTGGG 30
(2) INFORMATION POUR LA SEQ ID NO: 27:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce AttP Nhe+
   (xi) DESCRIPTION DE LA SEQUENCE: SEQ ID NO: 27:
   CTAGCTAGCC GCGCTAATGC TCTGTTACAG 30
(2) INFORMATION POUR LA SEQ ID NO: 28:
   (i) CARACTERISTIQUES DE LA SEQUENCE:
      (A) LONGUEUR: 29 paires de bases
      (B) TYPE: acide nucléique
      (C) NOMBRE DE BRINS: simple
      (D) CONFIGURATION: linéaire
   (ii) TYPE DE MOLECULE : Oligonucléptide de sythèse amorce AttP Nhe-
   (xi) DESCRIPTION DE LA SEQUENCE: SEQ ID NO: 28:
   CTAGCTAGCA TCAAATAATG ATTTTATTT 29
(2) INFORMATION POUR LA SEQ ID NO: 29:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Amb PCR
   (xi) DESCRIPTION DE LA SEQUENCE: SEQ ID NO: 29:
   GCTCTAGACT AACTAGTTTT GTCACAAGAT TTG 33
(2) INFORMATION POUR LA SEQ ID NO: 30:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Pro Cm Kpn+
   (xi) DESCRIPTION DE LA SEQUENCE: SEQ ID NO: 30:
   GGGGTACCGA ATAAATACCT GTGA 24
(2) INFORMATION POUR LA SEQ ID NO: 31:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE : Oligonucléotide de synthèse : amorce Pro Cm Kpn-
   (xi) DESCRIPTION DE LA SEQUENCE: SEQ ID NO: 31.
   GGGGTACCAA AAAATACGCC CGGTA 25